# FASCICULE DE BREVET EUROPEEN

(11) **EP 0 772 581 B1**
(45) Date de publication et mention de la délivrance du brevet: **15.09.1999**
(21) Numéro de dépôt: 95925884.9
(22) Date de dépôt: 13.07.1995
(51) Int. Cl.: C07C 55/14, C07C 51/42, C07C 51/31

(54) **PROCEDE DE RECYCLAGE DU CATALYSEUR DANS L'OXYDATION DIRECTE DU CYCLOHEXANE EN L'ACIDE ADIPIQUE**
VERFAHREN ZUR KATALYSATORREZIRKULATION IN DER DIREKTEN OXIDATION VON CYCLOHEXAN ZUR ADIPINSÄURE
METHOD OF RECYCLING A CATALYST IN A REACTION INVOLVING THE DIRECT OXIDATION OF CYCLOHEXANE INTO ADIPIC ACID

(30) Priorité: 21.07.1994 FR 9409253
(43) Date de publication de la demande: 14.05.1997
(73) Titulaire: RHODIA FIBER & RESIN INTERMEDIATES, 92408 Courbevoie Cédex (FR)
(72) Inventeur: COSTANTINI, Michel, F-69003 Lyon (FR); FACHE, Eric, F-69100 Villeurbanne (FR); NIVERT, Daniel, F-38200 Seyssuel (FR)
(74) Mandataire: Vignally, Noel
(86) Numéro de dépôt international: FR9500944
(87) Numéro de publication internationale: WO9603365

(56) Documents cités:
- FR-A- 2 386 346

## Description

La présente invention concerne le domaine de l'oxydation en une étape du cyclohexane en acide adipique, à l'aide d'un gaz contenant de l'oxygène, en phase liquide et en présence d'un catalyseur contenant du cobalt.

L'oxydation directe du cyclohexane en acide adipique est un procédé qui a été travaillé depuis longtemps, notamment en raison des avantages évidents qu'il y aurait à convertir le cyclohexane en acide adipique, en une seule étape et sans mettre en oeuvre un oxydant tel que l'acide nitrique, ce composé générant des oxydes d'azote qu'il faut ensuite traiter pour éviter toute pollution.

Ainsi le brevet américain US-A-2 223 493, publié en décembre 1940, décrit l'oxydation d'hydrocarbures cycliques en diacides correspondants, en phase liquide comportant généralement de l'acide acétique, à une température d'au moins 60°C, à l'aide d'un gaz contenant de l'oxygène et en présence d'un catalyseur d'oxydation tel qu'un composé du cobalt. Ce brevet prévoit un séparation de l'acide adipique formé par cristallisation, mais n'enseigne rien sur la manière de recycler le catalyseur, dans une nouvelle opération d'oxydation, ni a fortiori sur l'activité qu'aurait un catalyseur recyclé une ou plusieurs fois.

La demande de brevet WO-A-94/07833 décrit un procédé similaire, en précisant que le solvant représente moins que 1,5 mole par mole d'hydrocarbure cyclique, que ledit solvant comprend un acide organique n'ayant que des atomes d'hydrogène primaires ou secondaires et que la réaction est conduite en présence d'au moins 0,002 mole de catalyseur à base de cobalt pour 1000 g de mélange réactionnel. En fin de réaction, le diacide formé est isolé.

La demande de brevet WO-A-94/07834, déposée le même jour que la précédente, décrit également le même procédé, mais développe les phases de traitement du mélange réactionnel final. Ce traitement consiste à séparer le diacide formé, en refroidissant le mélange pour provoquer la précipitation dudit diacide, à séparer par filtration le diacide de deux phases liquides, une non-polaire qui est recyclée, une polaire qui est également recyclée après une éventuelle hydrolyse et une séparation d'une quantité supplémentaire de diacide.

Ces différents brevets présentent des solutions permettant d'oxyder en une étape le cyclohexane en acide adipique avec une sélectivité industriellement acceptable, mais ils ne traitent pas du problème particulier de la désactivation progressive et relativement rapide du catalyseur, lors de son recyclage.

En effet, lorsque le mélange réactionnel issu de la réaction d'oxydation du cyclohexane est refroidi pour faire cristalliser une partie de l'acide adipique, puis filtré pour séparer cet acide adipique précipité, le filtrat ainsi obtenu contient le catalyseur, de l'acide adipique résiduel, les sous-produits de la réaction (notamment acide glutarique, acide succinique, cyclohexanol, cyclohexanone, acide hydroxycaproïque, esters de cyclohexyle), le cyclohexane n'ayant pas réagi, le solvant acide acétique, l'eau formée.

Une partie de ces divers composés a certainement une influence sur la désactivation du catalyseur. Dans le procédé décrit dans la demande de brevet WO-A-94/07834 la plus grande partie de ces différents composés est recyclée à l'oxydation, éventuellement après ajout de quantités complémentaires de cyclohexane et éventuellement après séparation d'une quantité supplémentaire d'acide adipique restant dans le mélange. Il s'avère que lorsque le catalyseur et les différents sous-produits sont recyclés dans une nouvelle réaction d'oxydation, on observe une désactivation relativement rapide du catalyseur. Ainsi, il est indiqué dans les différents exemples de WO-A-94/07834 que la vitesse de formation de l'acide adipique diminue de 26 % à 43 % après quatre tours du catalyseur.

Un des objets de la présente invention est donc de permettre le recyclage du catalyseur au cobalt avec une désactivation faible ou nulle.

Afin d'atteindre ce but, le procédé de l'invention comporte une étape de traitement du mélange réactionnel obtenu lors de l'oxydation directe du cyclohexane en acide adipique, consistant en une extraction d'au moins une partie des acides glutarique et succinique formés dans la réaction.

Un premier objet de l'invention consiste tout d'abord en un procédé de recyclage d'un catalyseur contenant du cobalt, dans une réaction d'oxydation directe du cyclohexane en acide adipique, dans un solvant comprenant au moins un acide carboxylique aliphatique ne possédant que des atomes d'hydrogène primaires ou secondaires, par un gaz contenant de l'oxygène, ledit procédé de recyclage étant caractérisé :
- en ce que le mélange réactionnel provenant d'une opération antérieure d'oxydation du cyclohexane en acide adipique est traité pour éliminer les composés les plus volatils, après éventuellement la cristallisation et la séparation d'au moins une partie de l'acide adipique qu'il contient,
- en ce que le résidu obtenu est extrait à l'aide d'un solvant, choisi parmi les cétones, les alcools, les esters, leurs différents mélanges ou les mélanges d'hydrocarbure et d'acide carboxylique, solvant capable de dissoudre la totalité ou une grosse partie des diacides que contient ledit résidu,
- en ce que le résidu d'extraction ainsi obtenu, contenant la plus grosse partie du catalyseur au cobalt, est utilisé dans une nouvelle opération d'oxydation du cyclohexane en acide adipique, après ajout des compléments nécessaires en cyclohexane, en acide carboxylique et le cas échéant en catalyseur au cobalt.

Par l'expression "grosse partie", on entend dans le présent texte au moins 50 % en poids de la quantité totale du ou des composés considérés.

Le traitement du mélange réactionnel peut se faire commodément par distillation, sous pression atmosphérique ou sous pression réduite, des composés les plus volatils qui sont notamment le cyclohexane n'ayant pas réagi, l'acide carboxylique servant de solvant dans la réaction d'oxydation, l'eau formée et certains composés intermédiaires comme le cyclohexanol et la cyclohexanone. Comme indiqué auparavant, ce traitement peut être précédé de la cristallisation d'une partie ou de la totalité de l'acide adipique, par refroidissement du mélange réactionnel, et de sa séparation, par exemple par filtration ou centrifugation.

Les cétones qui peuvent servir à l'extraction du catalyseur du résidu obtenu sont notamment l'acétone, la méthyléthylcétone, la cyclohexanone, la cyclohexanone étant préférée car elle peut le cas échéant être réutilisée dans d'autres phases du procédé, par exemple être recyclée en oxydation.

Les alcools qui peuvent servir à l'extraction du catalyseur du résidu obtenu sont notamment le propanol-1, le propanol-2, le butanol-1, le butanol-2, le tertiobutanol, le cyclohexanol, le cyclohexanol étant préféré car il peut le cas échéant être réutilisé dans d'autres phases du procédé, par exemple être recyclé en oxydation.

Les esters qui peuvent servir à l'extraction du catalyseur du résidu obtenu sont notamment les esters dérivant des alcools indiqués précédemment avec des acides carboxyliques aliphatiques ne possédant que des atomes d'hydrogène primaires ou secondaires, tels que ceux qui sont mis en oeuvre dans la réaction d'oxydation du cyclohexane.

Des mélanges de plusieurs de ces solvants d'extraction peuvent bien sûr être mis en oeuvre, notamment des mélanges de cyclohexanol et de cyclohexanone (parfois appelés olone). D'autres mélanges de solvants peuvent également convenir, par exemples des mélanges d'hydrocarbures aliphatiques ou cycloaliphatiques et d'acides carboxyliques, qui seront définis ci-après.

Un deuxième objet de l'invention consiste également en un procédé de recyclage d'un catalyseur contenant du cobalt, dans une réaction d'oxydation directe du cyclohexane en acide adipique, par un gaz contenant de l'oxygène, ledit procédé de recyclage étant caractérisé :
- en ce que le mélange réactionnel, provenant d'une opération antérieure d'oxydation du cyclohexane en acide adipique, dont au moins une partie des produits intermédiaires d'oxydation, tels que notamment le cyclohexanol et la cyclohexanone, du solvant acide carboxylique et de l'eau a été séparée et dont au moins une partie de l'acide adipique formé a été récupérée par cristallisation, est soumis à au moins une extraction à l'aide d'au moins un cosolvant ou d'un mélange comprenant un cosolvant et un acide carboxylique,
- en ce que l'on sépare d'une part un mélange contenant au moins une partie du catalyseur au cobalt, une partie de l'acide carboxylique et éventuellement des quantités résiduelles d'autres composés et d'autre part une solution contenant le cosolvant et au moins une partie des acides glutarique et succinique formés dans la réaction d'oxydation, ainsi que de l'acide carboxylique,
- et en ce que le mélange contenant au moins une partie du catalyseur au cobalt est utilisé dans une nouvelle opération d'oxydation du cyclohexane en acide adipique, éventuellement après l'ajout d'un appoint en catalyseur au cobalt.

Les cosolvants qui peuvent être mis en oeuvre dans le procédé selon le deuxième objet de l'invention sont généralement choisis parmi les hydrocarbures, surtout aliphatiques et cycloaliphatiques, les cétones et les alcools.

Parmi les hydrocarbures, on peut citer l'hexane, l'heptane, l'octane, le nonane, le décane, l'undécane, le dodécane, le cyclohexane.

Parmi les cétones, on peut utiliser celles qui ont été citées pour l'extraction du résidu dans le procédé selon le premier objet de l'invention, la cyclohexanone étant préférée.

Parmi les alcools, on peut utiliser ceux qui ont été cités pour l'extraction du résidu dans le procédé selon le premier objet de l'invention, le cyclohexanol étant préféré.

Il est préférable d'utiliser le cyclohexane comme cosolvant dans le procédé selon le deuxième objet de l'invention, car cela facilite le traitement des différentes solutions obtenues au cours du procédé et leur recyclage éventuel dans la réaction d'oxydation.

Le mélange réactionnel brut qui, après certaines opérations de séparation, est mis en oeuvre dans le procédé de l'invention, provient de l'oxydation connue en soi du cyclohexane, par un gaz contenant de l'oxygène, en milieu liquide comprenant un acide carboxylique et en présence d'un catalyseur contenant du cobalt.

Pour la préparation de ce mélange réactionnel brut, on peut se référer aux procédés décrits dans l'art antérieur notamment dans US-A-2 223 493 précité. Ainsi le rapport pondéral initial cyclohexane /acide carboxylique peut se situer par exemple entre 0,1/1 et 10/1 et de préférence entre 0,5/1 et 3/1. Le catalyseur au cobalt comprend de préférence un composé du cobalt soluble dans le milieu réactionnel, choisi par exemple parmi les carboxylates de cobalt, comme de préférence l'acétate de cobalt tétrahydraté, le chlorure de cobalt, le bromure de cobalt, le nitrate de cobalt.

La quantité de catalyseur, exprimée en pourcentage pondéral de cobalt par rapport au mélange réactionnel, se situe généralement entre 0,01 % et 5 % et de préférence entre 0,05 % et 2 %, sans que ces valeurs soient critiques. Il s'agit cependant d'avoir une activité suffisante tout en n'utilisant pas des quantités trop importantes d'un catalyseur qu'il faut ensuite séparer du mélange réactionnel final et recycler.

Le catalyseur, outre le cobalt, peut également comporter d'autres composés à base de métaux tels que le manganèse et/ou le cuivre et/ou le cérium et/ou le vanadium.

Il est avantageux de mettre en oeuvre également un composé initiateur de la réaction d'oxydation, tel que par exemple une cétone ou un aldéhyde. La cyclohexanone qui est un intermédiaire réactionnel est tout particulièrement indiquée. Généralement l'initiateur représente de 0,01 % à 20 % en poids du poids du mélange réactionnel mis en oeuvre, sans que ces proportions aient une valeur critique. L'initiateur est surtout utile lors du démarrage de l'oxydation et lorsque l'on réalise l'oxydation du cyclohexane à une température inférieure à 120°C. Il peut être introduit dès le début de la réaction.

L'acide carboxylique servant de solvant dans la réaction d'oxydation du cyclohexane est plus particulièrement un acide carboxylique aliphatique saturé ayant de 2 à 9 atomes de carbone et n'ayant que des atomes d'hydrogène primaires ou secondaires.

L'acide acétique est utilisé de préférence comme solvant de la réaction d'oxydation du cyclohexane. Dans la suite de la présente description, on se référera par commodité à l'acide acétique comme acide carboxylique utilisé dans les diverses étapes du procédé.

L'oxydation peut également être mise en oeuvre en présence d'eau introduite dès le stade initial du procédé.

La réaction d'oxydation du cyclohexane est généralement effectuée à une température de 60°C à 180°C et de préférence de 70°C à 120°C.

La pression n'est pas un paramètre critique de la réaction et se situe généralement entre 10 kPa (0,1 bar) et 10000 kPa (100 bars).

Avant de réaliser l'opération d'extraction selon l'invention, le mélange réactionnel brut issu de l'oxydation du cyclohexane est soumis à différentes opérations de séparation de certains de ses constituants.

Selon une première variante du procédé selon le deuxième objet de l'invention, on peut soumettre tout d'abord le mélange réactionnel brut à un refroidissement à une température de 16°C à 30°C par exemple, ce qui occasionne la cristallisation d'au moins une partie de l'acide adipique formé. On obtient ainsi un milieu triphasique comprenant une phase solide constituée essentiellement d'acide adipique, une phase liquide cyclohexanique supérieure contenant essentiellement le cyclohexane n'ayant pas réagi et une phase liquide acétique inférieure contenant essentiellement l'acide acétique, l'eau formée, de l'acide adipique, les intermédiaires d'oxydation du cyclohexane tels que le cyclohexanol, la cyclohexanone, l'acide hydroxycaproïque, les sous-produits tels que les acides glutarique et succinique et le catalyseur au cobalt. Le milieu obtenu par refroidissement du mélange réactionnel peut le cas échéant être biphasique, c'est-à-dire ne comporter que l'acide adipique précipité et la phase acétique, si la transformation du cyclohexane au cours de l'oxydation est complète ou presque complète.

Après filtration ou centrifugation du solide, on procède s'il y a lieu à la séparation par décantation des deux phases liquides constituant le filtrat ou le centrifugat: la phase cyclohexanique, qui contient de faibles quantités de produits d'oxydation du cyclohexane, peut être recyclée dans une nouvelle réaction d'oxydation.

Il peut aussi être avantageux de procéder, préalablement à l'opération de cristallisation de l'acide adipique, à une concentration du mélange réactionnel ; on peut alors retrouver, lors de la précipitation de l'acide adipique, une seule phase liquide acétique.

Selon une deuxième variante du procédé selon le deuxième objet de l'invention, on peut soutirer à chaud le mélange réactionnel brut final, par exemple à une température pouvant atteindre 75 °C. Le mélange réactionnel décante alors en deux phases liquides : une phase cyclohexanique supérieure contenant essentiellement le cyclohexane n'ayant pas réagi et une phase liquide acétique inférieure contenant essentiellement l'acide acétique, l'acide adipique, l'eau formée, les intermédiaires d'oxydation du cyclohexane tels que le cyclohexanol, la cyclohexanone, l'acide hydroxycaproïque, les sous-produits tels que les acides glutarique et succinique et le catalyseur au cobalt.

Comme dans la première variante, on procède à la séparation par décantation des deux phases liquides : la phase cyclohexanique, qui contient de faibles quantités de produits d'oxydation du cyclohexane, peut être recyclée dans une nouvelle réaction d'oxydation.

La remarque faite précédemment pour la première variante est également valable pour les autres variantes : si le cyclohexane mis en oeuvre à l'oxydation est pratiquement tout transformé, il peut ne pas y avoir deux phases liquides, mais une phase acétique unique.

La phase acétique, après une éventuelle concentration, est ensuite refroidie à une température de 16°C à 30°C par exemple, ce qui occasionne la cristallisation d'au moins une partie de l'acide adipique formé, qui est ensuite séparé par filtration ou centrifugation. Cet acide adipique peut être purifié par recristallisation dans un solvant approprié, qui peut avantageusement être l'acide acétique ou l'eau. Lorsque l'acide acétique sert de solvant de recristallisation, il peut ensuite être rajouté à la phase acétique obtenue précédemment.

Selon une troisième variante du procédé selon le deuxième objet de l'invention, on peut pendant la réaction d'oxydation distiller un mélange azéotropique eau/cyclohexane, puis après décantation de ce mélange réintroduire, si on le souhaite, le cyclohexane dans le réacteur. Cela permet d'éliminer au moins une partie de l'eau du mélange réactionnel. Le traitement du mélange réactionnel brut final peut se faire ensuite selon la première variante décrite précédemment, c'est-à-dire refroidissement pour faire précipiter l'acide adipique, filtration ou centrifugation. L'élimination de l'eau a pour conséquence d'éviter l'éventuelle décantation de la partie liquide en deux phases distinctes. La phase liquide unique obtenue est alors traitée comme la phase acétique.

La phase acétique, obtenue dans l'une ou l'autre de ces trois variantes de traitement du mélange réactionnel brut final, est soumise à une extraction par le cyclohexane, soit telle qu'elle est obtenue après décantation (notamment dans le cas où l'eau en a été éliminée), soit de préférence après avoir été concentrée par chauffage à une température de 30°C à 80° sous pression réduite. Cette concentration permet d'éliminer une partie de l'acide acétique, au moins la majeure partie de l'eau et une partie au moins des composés légers qui peuvent être présents, tels que du cyclohexane, du cyclohexanol ou de la cyclohexanone. Les composés ainsi séparés de la phase acétique peuvent être recyclés dans l'étape d'oxydation du cyclohexane, soit en totalité, soit après séparation d'au moins une partie de l'eau qu'ils contiennent. Généralement la concentration réduit la phase acétique à un volume représentant de 80 % à 10 % de son volume initial, ces valeurs n'étant données qu'à titre d'exemples. Une variante consiste à concentrer à sec la phase acétique, c'est-à-dire à éliminer la totalité de l'acide acétique qu'elle contient.

La concentration partielle de la phase acétique peut également permettre, si elle est suivie par un refroidissement, dans les conditions indiquées précédemment pour la cristallisation de l'acide adipique, de faire précipiter une quantité supplémentaire d'acide adipique.

L'extraction de la phase acétique, concentrée ou non, est réalisée soit avec le seul cyclohexane, soit avec des mélanges cyclohexane/acide acétique. Le point essentiel est cependant que l'ensemble constitué de la phase acétique soumise à l'extraction et du cyclohexane ait globalement un rapport pondéral cyclohexane/acide acétique compris entre 1/1 et 50/1 et de préférence entre 2/1 et 15/1. Cela signifie que, selon la composition de la phase acétique obtenue après les différents traitements du mélange réactionnel décrits précédemment, l'acide acétique nécessaire pour réaliser l'extraction avec un mélange cyclohexane/acide acétique de la composition ainsi définie sera constitué pour une part ou pour la totalité par l'acide acétique contenu dans la phase acétique ou sera introduit avec le cyclohexane dans le cas où l'acide acétique de ladite phase acétique aura été éliminé.

L'extraction peut être réalisée en une ou plusieurs fois ou dans le cadre d'un procédé en continu par les techniques industrielles habituelles. Elle peut être conduite à une température allant jusqu'à la température d'ébullition du ou des solvants utilisés. Généralement on opère entre 10°C et 80°C et de préférence entre 50°C et 80°C.

L'opération d'extraction conduit d'une part à une solution qui contient au moins une partie des acides glutarique et succinique que l'on souhaite séparer, ainsi que des quantités résiduelles d'autres sous-produits qui peuvent encore demeurer, tels que des lactones, des esters et des produits de sur-oxydation, et d'autre part à un mélange contenant essentiellement le catalyseur au cobalt. Ce mélange est généralement séparé par décantation.

Le catalyseur au cobalt ainsi séparé est recyclé dans une nouvelle réaction d'oxydation du cyclohexane, éventuellement après l'ajout d'un appoint pour compenser les pertes subies au cours des différents traitements du mélange réactionnel issu de l'oxydation du cyclohexane.

Ce catalyseur demeure aussi actif que le catalyseur neuf mis en oeuvre dans la première opération d'oxydation du cyclohexane et il peut ainsi être recyclé un grand nombre de fois sans diminution importante de son activité et de la sélectivité de la réaction en acide adipique.

Un troisième objet de l'invention consiste en un procédé continu d'oxydation du cyclohexane en acide adipique, à l'aide d'un gaz contenant de l'oxygène , en milieu liquide comprenant un acide carboxylique comme solvant et en présence d'un catalyseur contenant au moins du cobalt, caractérisé en ce qu'il comporte les étapes suivantes:
a) l'oxydation proprement dite du cyclohexane en acide adipique,
b) le mélange réactionnel provenant de l'oxydation du cyclohexane en acide adipique est traité pour éliminer les composés les plus volatils, après éventuellement la cristallisation et la séparation d'au moins une partie de l'acide adipique qu'il contient,
c) le résidu obtenu est extrait à l'aide d'un solvant, choisi parmi les cétones, les alcools, les esters, leurs différents mélanges ou les mélanges d'hydrocarbure et d'acide carboxylique, solvant capable de dissoudre la totalité ou une grosse partie des diacides que contient ledit résidu,
d) le résidu d'extraction ainsi obtenu, contenant la plus grosse partie du catalyseur au cobalt, est utilisé dans une nouvelle opération d'oxydation du cyclohexane a).

Chacune des différentes étapes de ce procédé continu a été détaillée précédemment et l'on peut se référer à cette description pour les modalités particulières de mise en oeuvre du procédé.

Un quatrième objet de l'invention consiste en un procédé continu d'oxydation du cyclohexane en acide adipique, à l'aide d'un gaz contenant de l'oxygène , en milieu liquide comprenant de l'acide acétique comme solvant et en présence d'un catalyseur contenant au moins du cobalt, caractérisé en ce qu'il comporte les étapes suivantes :
a) l'oxydation proprement dite du cyclohexane en acide adipique,
b)
   soit : b1) le refroidissement du mélange réactionnel final brut, éventuellement après une concentration, afin de faire cristalliser au moins une partie de l'acide adipique, la séparation dudit acide adipique par filtration ou centrifugation, puis si nécessaire la séparation par décantation du filtrat ou du centrifugat obtenu en une phase cyclohexanique et une phase acétique,
   soit: b2) le soutirage à chaud du mélange réactionnel final brut, ledit mélange réactionnel étant biphasique ou monophasique, son éventuelle séparation par décantation en une phase cyclohexanique et une phase acétique, le refroidissement de la phase acétique décantée ou le cas échéant de la phase acétique unique afin de faire cristalliser au moins une partie de l'acide adipique, la séparation par filtration ou centrifugation dudit acide adipique et de la phase acétique,
   soit: b3) l'élimination de l'eau du mélange réactionnel pendant l'étape a) d'oxydation, par distilation de l'azéotrope cyclohexane/eau et réintroduction éventuelle dans le réacteur du cyclohexane distillé, puis traitement du mélange réactionnel final brut selon la variante b1), ledit traitement conduisant dans ce cas à une phase liquide unique qui est traitée pour la suite du procédé comme une phase acétique,
c) la concentration de la phase acétique pour en éliminer au moins la majeure partie de l'eau, si cela n'a pas été fait dans la variante b3),
d) l'extraction de la phase acétique à l'aide de cyclohexane ou de mélange cyclohexane/acide acétique en quantité telle que dans l'ensemble cyclohexane/phase acétique le rapport pondéral global cyclohexane/acide acétique soit compris entre 1/1 et 50/1 et de préférence entre 2/1 et 15/1,
e) la séparation d'une solution cyclohexanique contenant au moins une partie des acides glutarique et succinique, d'une part, et d'un mélange contenant essentiellement le catalyseur au cobalt, d'autre part,
f) le recyclage du mélange contenant le catalyseur au cobalt dans une nouvelle réaction d'oxydation a).

Chacune des différentes étapes de ce procédé continu a été détaillée précédemment et l'on peut se référer à cette description pour les modalités particulières de mise en oeuvre du procédé.

Les exemples qui suivent illustrent la présente invention.

### EXEMPLES 1 A 5

Dans un autoclave de 1,5 litre chemisé en titane et équipé d'une double turbine six pales et de diverses ouvertures pour l'introduction des réactifs et des fluides ou pour l'évacuation des produits de la réaction et des fluides, que l'on a préalablement purgé à l'azote, on charge à température ambiante :
- acétate de cobalt tétrahydraté : 4,0 g (16 mmol)
- acide acétique : 359 g (5,98 mol)
- cyclohexane : 289,7 (3,45 mol)
- acétaldéhyde : 1,2 g (27,3 mmol).

Après fermeture de l'autoclave, la pression d'azote est portée à 20 bar, l'agitation est mise en route à 800 tours/min et la température est amenée à 102°C en 29 min. L'azote est alors remplacé par 20 bar d'air appauvri (5,85 % d'oxygène). Le débit gazeux de sortie est réglé à 250 litres/heure.

Après une induction de 10 min environ, pendant laquelle il n'y a pas de consommation d'oxygène, la température s'élève brutalement à 106°C et l'oxygène commence à être consommé. Quand la teneur en oxygène de l'air à la sortie de l'autoclave atteint 1 %, l'alimentation en air appauvri à 5,85 % d'oxygène est remplacée par une alimentation en air à 11,35 % d'oxygène. La teneur en oxygène à la sortie du réacteur reste inférieure à 1 % durant la totalité de l'essai. La température moyenne dans l'autoclave est maintenue à 106-107°C.

Lorsque 50 litres d'oxygène ont été consommés, la vanne de sortie ainsi que l'alimentation en air sont fermées. Dans le même temps, la température dans l'autoclave est progressivement ramenée à 75°C.

Le mélange réactionnel est alors récupéré à l'aide d'une vanne de prélèvement reliée à un tube plongeant, puis il est rapidement refroidi vers 20°C.

On obtient un milieu triphasique constitué d'acide adipique brut ayant précipité (49 g), d'une phase acétique (451,4 g) et d'une phase cyclohexanique (143,2 g). L'autoclave est rincé avec de l'acide acétique et ce lavage est rajouté à la phase acétique. Le contenu d'un piège (5,5 g) placé après un réfrigérant de condensation est ajouté à la phase cyclohexanique.

Par recristallisation dans l'acide acétique, on obtient 294,5 mmol d'acide adipique recristallisé, ainsi que 38,5 mmol d'acide adipique, 1,6 mmol d'acide glutarique et 1,2 mmol d'acide succinique dissous. Environ 5 % du catalyseur, qui ont été entrainés par la précipitation de l'acide adipique, sont récupérés dans l'acide acétique de recristallisation et cette solution acétique est rajoutée à la phase acétique.

Les phases acétique et cyclohexanique sont dosées par chromatographie en phase vapeur.

Les résultats de l'exemple 1 ainsi que des exemples ou essais comparatifs qui suivent sont exprimés de la manière suivante :
- taux de conversion (TT) du cyclohexane : % molaire du cyclohexane converti dans les différents composés du mélange réactionnel final ;
- sélectivité (RT) en produit P : moles de produit P x 100/ moles de cyclohexane converties ;
- linéarité en diacides formés (Lté): moles acide adipique formé x 100/ somme des moles des acides adipique, glutarique et succinique formés.

La phase acétique contient la quasi totalité des acides et des lactones formés et la très grande majorité du cyclohexanol, de la cyclohexanone et de l'acétate de cyclohexyle formés, ainsi que l'eau et la quasi-totalité du catalyseur.

La phase acétique est concentrée par chauffage à 50°C, sous pression réduite (4 kPa) pour éliminer l'eau, une partie de l'acide acétique, du cyclohexane, du cyclohexanol et de la cyclohexanone, jusqu'à obtenir une solution contenant 110 g d'acide acétique et 35 g de produits ( essentiellement les diacides et le catalyseur au cobalt).

Cette phase acétique concentrée est traitée avec 1300 g de cyclohexane à 70°C. Il apparaît un précipité rouge et un liquide sumageant incolore qui est éliminé à chaud (60°C).

Le sumageant contient l'essentiel des produits d'oxydation (diacides, cyclohexanol, cyclohexanone, esters, lactones) tandis que le précipité contient plus de 95 % du cobalt (quantité déterminée par dosage sur un échantillon) qui est renvoyé au réacteur avec les appoints nécessaires en cobalt, cyclohexane et acide acétique pour retrouver les proportions indiquées précédemment pour le premier essai.

On effectue 4 recyclages successifs du catalyseur (exemples 2 à 5).

Les résultats obtenus avec les exemples 1à 5 sont rassemblés dans le tableau 1 ci-après.

Dans ce tableau les abréviations nouvelles suivantes sont utilisées :
- AdOH pour acide adipique
- olone pour le mélange cyclohexanol/cyclohexanone
- O2 pour la quantité maximale d'oxygène consommée en litres/heure (cette valeur est représentative de la vitesse maximale d'oxydation)
- Pté en AdOH pour productivité en acide adipique formé exprimée en g/l.h.

**Tableau 1**

| Exemples | Durée de réaction | O2 en l/min | TT % du cyclohexane | RT % en AdOH | Pté en AdOH | RT % en olone | Lté % |
|---|---|---|---|---|---|---|---|
| Exemple 1 | 125 min | 0,45 | 23,2 | 68,5 | 54,7 | 15,2 | 87,7 |
| Exemple 2 | 130 min | 0,45 | 23,9 | 67,9 | 53,8 | 14,9 | 86,9 |
| Exemple 3 | 133 min | 0,44 | 23,5 | 68,3 | 52,0 | 14,5 | 87,1 |
| Exemple 4 | 130 min | 0,44 | 22,9 | 67,9 | 51,5 | 14,8 | 86,8 |
| Exemple 5 | 130 min | 0,44 | 24,6 | 68,3 | 55,7 | 13,9 | 86,4 |

On constate que l'activité catalytique, les sélectivités en acide adipique et en olone (mélange de composés adipogènes) et la linéarité sont conservées sur 5 oxydations successives.

### ESSAIS COMPARATIFS a A e

Le premier essai d'oxydation est réalisé avec les mêmes quantités de réactifs et de catalyseur et les mêmes conditions opératoires que l'exemple 1, mais le traitement du mélange réactionnel final est différent.

Comme dans l'exemple 1, la phase acétique contient la quasi totalité des acides et des lactones formés et la très grande majorité du cyclohexanol, de la cyclohexanone et de l'acétate de cyclohexyle formés, ainsi que l'eau et la quasi-totalité du catalyseur (environ 5 % du catalyseur, qui ont été entraînés par la précipitation de l'acide adipique, sont récupérés dans l'acide acétique utilisé pour la recristallisation de l'acide adipique et cette solution acétique est rajoutée à la phase acétique.

La phase acétique est concentrée par chauffage à 50°C, sous pression réduite (4 kPa) pour éliminer l'eau, une partie de l'acide acétique, du cyclohexane, du cyclohexanol et de la cyclohexanone, jusqu'à obtenir une solution contenant 110 g d'acide acétique et 35 g de produits ( essentiellement les diacides et le catalyseur au cobalt).

Cette solution concentrée acétique est renvoyée au réacteur avec les appoints nécessaires en cobalt, cyclohexane et acide acétique pour retrouver les proportions indiquées précédemment pour le premier essai a.

On effectue 4 recyclages successifs du catalyseur (essais comparatifs b, c, d, e).

Les résultats obtenus avec les essais a à e sont rassemblés dans le tableau 2 ci-après.

**Tableau 2**

| Essai comparatif | Durée de réaction | O2 en l/min | TT % du cyclohexane | RT % en AdOH | Pté en AdOH | RT % en olone | Lté % |
|---|---|---|---|---|---|---|---|
| Essai a | 122 min | 0,46 | 21,7 | 65,4 | 50,1 | 15,0 | 86,8 |
| Essai b | 125 min | 0,44 | 21,3 | 69,0 | 50,6 | 9,1 | 84,7 |
| Essai c | 130 min | 0,36 | 13,8 | 71,0 | 32,5 | 7,0 | 84,3 |
| Essai d | 181 min | 0,30 | 16,2 | 71,1 | 27,4 | 7,6 | 87,7 |
| Essai e | 223 min | 0,21 | 15,2 | 69,7 | 20,5 | 9,1 | 85,3 |

On constate que la sélectivité globale en acide adipique et olone et la linéarité sont conservées sur 5 oxydations successives. Par contre si l'activité est conservée au cours du premier recyclage du catalyseur, on observe une désactivation progressive dudit catalyseur.

En outre, l'acide adipique brut qui précipite lors du refroidissement du mélange réactionnel final est de plus en plus souillé par les autres produits d'oxydation dont la concentration augmente au fil des recyclages et cet acide adipique précipité entraîne de plus en plus de cobalt.

### EXEMPLE 6

Une solution, homogène à 100°C, contenant 120 g d'acide acétique, 3,7 g d'acide succinique (31,6 mmol), 7,3 g d'acide glutarique (55,3 mmol) et 29,2 g d'acide adipique (200 mmol) ainsi que 4 g d'acétate de cobalt tétrahydraté (16 mmol), est progressivement ramenée à température ambiante.

On sépare par filtration un solide contenant 21 g d'acide adipique et on récupère un filtrat constitué de 116 g d'acide acétique, de 3,0 g d'acide succinique (25,4 mmol), de 7,0 g d'acide glutarique (53 mmol) et de 8,2 g d'acide adipique (56 mmol) ainsi que de 4 g d'acétate de Co tétrahydraté (16 mmol).

Ce filtrat est concentré à sec, puis traité par 2 fois 50 ml d'acétone à chaud (56°C).

La répartition des composés entre l'extrait acétonique et le résidu solide restant est indiquée dans le tableau 3 ci-après.

**Tableau 3**

| Composés | Filtrat à sec | Extrait acétonique | Résidu solide après extraction |
|---|---|---|---|
| acide succinique | 25,4 mmol | 18 mmol (70,9 %) | 7,1 mmol (28 %) |
| acide glutarique | 53 mmol | 36,4 mmol (68,7 %) | 16,1 mmol (30,4 %) |
| acide adipique | 56 mmol | 45,1 mmol (80,5 %° | 10,7 mmol (19,1 %) |
| cobalt | 16 mmol | <0,016 mmol (<0,1%) | 15,9 mmol (99,4 %) |

### EXEMPLE 7

Une solution de même composition que celle de l'exemple 6 est traitée de la même manière pour séparer par cristallisation et filtration la majeure partie de l'acide adipique.

Le filtrat obtenu est constitué de 115 g d'acide acétique, de 3,7 g d'acide succinique (31 mmol), de 7,3 g d'acide glutarique (55 mmol) et de 8,8 g d'acide adipique (60 mmol) ainsi que de 4 g d'acétate de Co tétrahydraté (16 mmol).

Ce filtrat est concentré à sec, puis traité par 5 fois 100 ml d"un mélange cyclohexanone/acide acétique (82/18 en poids par poids) à chaud (70°C).

La répartition des composés entre l'extrait du mélange de solvants et le résidu solide restant est indiquée dans le tableau 4 ci-après.

**Tableau 4**

| Composés | Filtrat à sec | Extrait solvants | Résidu solide après extraction |
|---|---|---|---|
| acide succinique | 31 mmol | 16,4 mmol (52,9 %) | 14,5 mmol (46,7 %) |
| acide glutarique | 55 mmol | 44,8 mmol (81,5 %) | 10,1 mmol (18,4 %) |
| acide adipique | 60 mmol | 42,9 mmol (71,5 %° | 17,1 mmol (28,5 %) |
| cobalt | 16 mmol | 0,9 mmol (5,6%) | 15 mmol (93,8 %) |

### EXEMPLE 8

Une solution, homogène à 100°C, contenant 120 g d'acide acétique, 3,4 g d'acide succinique, 6,3 g d'acide glutarique et 29,9 g d'acide adipique, ainsi que 4,1 g d'acétate de cobalt tétrahydraté, est progressivement ramenée à température ambiante.

On sépare par filtration un solide contenant 21,2 g d'acide adipique et on récupère un filtrat constitué de 116 g d'acide acétique, de 3,2 g d'acide succinique (27,1 mmol), de 6,2 g d'acide glutarique (47 mmol) et de 8,7 g d'acide adipique (60 mmol) ainsi que de 4,1 g d'acétate de Co tétrahydraté (16,4 mmol).

Ce filtrat est concentré en partie, pour donner une solution à chaud de 52 g environ. Cette solution concentrée chaude est traitée par coulée de 168,5 g d'acétone. La température du mélange se stabilise à 60°C. Le mélange est constitué par un solide et un liquide surnageant, qui sont séparés à chaud.

La répartition des composés entre le liquide sumageant et le résidu solide séparé est indiquée dans le tableau 5 ci-après.

**Tableau 5**

| Composés | Filtrat initial | Liquide surnageant | Résidu solide séparé |
|---|---|---|---|
| acide succinique | 27,1 mmol | 17,2 mmol (63,5 %) | 9,5 mmol (35 %) |
| acide glutarique | 47 mmol | 42,5 mmol (90,4 %) | 4,5 mmol (9,6 %) |
| acide adipique | 60 mmol | 55,5 mmol (92,5 %° | 4,4 mmol (7,3 %) |
| cobalt | 16 mmol | 0,25 mmol (1,5%) | 15 mmol (98,2 %) |

## Revendications

1. Procédé de recyclage d'un catalyseur contenant du cobalt, dans une réaction d'oxydation directe du cyclohexane en acide adipique, caractérisé en ce qu'il comporte une étape de traitement du mélange réactionnel obtenu lors de l'oxydation du cyclohexane en acide adipique, consistant en une extraction d'au moins une partie des acides glutarique et succinique formés dans la réaction.

2. Procédé selon la revendication 1 de recyclage d'un catalyseur contenant du cobalt, dans une réaction d'oxydation directe du cyclohexane en acide adipique, dans un solvant comprenant au moins un acide carboxylique aliphatique ne possédant que des atomes d'hydrogène primaires ou secondaires, par un gaz contenant de l'oxygène, ledit procédé de recyclage étant caractérisé :
- en ce que le mélange réactionnel provenant d'une opération antérieure d'oxydation du cyclohexane en acide adipique est traité par distillation pour éliminer les composés les plus volatils, après éventuellement la cristallisation et la séparation d'au moins une partie de l'acide adipique qu'il contient,
- en ce que le résidu obtenu est extrait à l'aide d'un solvant, choisi parmi les cétones, les alcools, les esters, leurs différents mélanges ou les mélanges d'hydrocarbure et d'acide carboxylique, solvant capable de dissoudre la totalité ou une grosse partie des diacides que contient ledit résidu,
- en ce que le résidu d'extraction ainsi obtenu, contenant la plus grosse partie du catalyseur au cobalt, est utilisé dans une nouvelle opération d'oxydation du cyclohexane en acide adipique, après ajout des compléments nécessaires en cyclohexane, en acide carboxylique et le cas échéant en catalyseur au cobalt.

3. Procédé selon la revendication 2, caractérisé en ce que le traitement du mélange réactionnel se fait par distillation, sous pression atmosphérique ou sous pression réduite, des composés les plus volatils qui sont notamment le cyclohexane n'ayant pas réagi, l'acide carboxylique servant de solvant dans la réaction d'oxydation, l'eau formée et certains composés intermédiaires comme le cyclohexanol et la cyclohexanone.

4. Procédé selon la revendication 2, caractérisé en ce que le solvant qui sert à l'extraction du catalyseur du résidu obtenu est choisi parmi l'acétone, la méthyléthylcétone, la cyclohexanone, le propanol-1, le propanol-2, le butanol-1, le butanol-2, le tertiobutanol, le cyclohexanol, les esters dérivant des alcools indiqués précédemment avec des acides carboxyliques aliphatiques ne possédant que des atomes d'hydrogène primaires ou secondaires, tels que ceux qui sont mis en oeuvre dans la réaction d'oxydation du cyclohexane, des mélanges de plusieurs de ces solvants d'extraction, des mélanges d'hydrocarbures aliphatiques ou cycloaliphatiques et d'acides carboxyliques.

5. Procédé selon la revendication 1 de recyclage d'un catalyseur contenant du cobalt, dans une réaction d'oxydation directe du cyclohexane en acide adipique, par un gaz contenant de l'oxygène, ledit procédé de recyclage étant caractérisé:
- en ce que le mélange réactionnel, provenant d'une opération antérieure d'oxydation du cyclohexane en acide adipique, dont au moins une partie des produits intermédiaires d'oxydation, tels que notamment le cyclohexanol et la cyclohexanone, du solvant acide carboxylique et de l'eau a été séparée et dont au moins une partie de l'acide adipique formé a été récupérée par cristallisation, est soumis à au moins une extraction à l'aide d'au moins un cosolvant ou d'un mélange comprenant un cosolvant et un acide carboxylique,
- en ce que l'on sépare d'une part un mélange contenant au moins une partie du catalyseur au cobalt, une partie de l'acide carboxylique et éventuellement des quantités résiduelles d'autres composés et d'autre part une solution contenant le cosolvant et au moins une partie des acides glutarique et succinique formés dans la réaction d'oxydation, ainsi que de l'acide carboxylique,
- et en ce que le mélange contenant au moins une partie du catalyseur au cobalt est utilisé dans une nouvelle opération d'oxydation du cyclohexane en acide adipique, éventuellement après l'ajout d'un appoint en catalyseur au cobalt.

6. Procédé selon la revendication 5, caractérisé en ce que le cosolvant mis en oeuvre est choisi parmi les hydrocarbures aliphatiques et cycloaliphatiques, les cétones et les alcools, et est de préférence le cyclohexane.

7. Procédé selon l'une des revendications 2 à 5, caractérisé en ce que l'acide carboxylique aliphatique utilisé est l'acide acétique.

8. Procédé selon l'une des revendications 5 à 7, caractérisé en ce que le mélange réactionnel brut est soumis à un refroidissement à une température de 16°C à 30°C pour occasionner la cristallisation d'au moins une partie de l'acide adipique formé, en conduisant ainsi soit à un milieu triphasique comprenant une phase solide constituée essentiellement d'acide adipique, une phase liquide cyclohexanique supérieure et une phase liquide acétique inférieure, soit à un milieu biphasique comprenant une phase solide constituée essentiellement d'acide adipique et une phase acétique, puis, après filtration ou centrifugation du solide, séparation par décantation des deux phases liquides, s'il y a lieu.

9. Procédé selon l'une des revendications 5 ou 8, caractérisé en ce que l'on procède, préalablement à l'opération de cristallisation de l'acide adipique, à une concentration du mélange réactionnel.

10. Procédé selon l'une des revendications 5 à 7, caractérisé en ce que l'on soutire à chaud le mélange réactionnel brut final, ledit mélange réactionnel étant alors biphasique ou monophasique, l'on procède si nécessaire à la séparation par décantation de deux phases liquides : une phase cyclohexanique supérieure et une phase liquide acétique inférieure, l'on refroidit à une température de 16°C à 30°C ladite phase acétique inférieure ou la phase acétique unique, pour occasionner la cristallisation d'au moins une partie de l'acide adipique formé, qui est ensuite séparé par filtration ou centrifugation de la phase acétique.

11. Procédé selon l'une des revendications 8 ou 10, caractérisé en ce que l'on purifie l'acide adipique par recristallisation dans un solvant approprié, tel que l'acide acétique ou l'eau, éventuellement ensuite rajouté à la phase acétique obtenue précédemment.

12. Procédé selon l'une des revendications 2 à 5, caractérisé en ce que l'on distille un mélange azéotropique eau/cyclohexane pendant la réaction d'oxydation, puis l'on réintroduit le cyclohexane dans le réacteur après décantation de ce mélange et l'on traite le mélange réactionnel brut final.

13. Procédé selon l'une des revendications 7 à 12, caractérisé en ce que la phase acétique, obtenue dans le traitement du mélange réactionnel brut final, est soumise à une extraction par le cyclohexane, soit telle qu'elle est obtenue après décantation, soit de préférence après avoir été concentrée par chauffage à une température de 30°C à 80° sous pression réduite.

14. Procédé selon la revendication 13, caractérisé en ce que la concentration de la phase acétique réduit celle-ci à un volume représentant de 80 % à 10 % de son volume initial.

15. Procédé selon la revendication 13, caractérisé en ce que l'on concentre à sec la phase acétique, c'est-à-dire que l'on élimine la totalité de l'acide acétique qu'elle contient.

16. Procédé selon l'une des revendications 13 à 15, caractérisé en ce que les composés séparés de la phase acétique lors de sa concentration sont recyclés dans l'étape d'oxydation du cyclohexane, soit en totalité, soit après séparation d'au moins une partie de l'eau qu'ils contiennent.

17. Procédé selon l'une des revendications 7 à 16, caractérisé en ce que l'extraction de la phase acétique, concentrée ou non, est réalisée soit avec le seul cyclohexane, soit avec des mélanges cyclohexane/acide acétique, de manière à ce que l'ensemble phase acétique soumise à l'extraction/cyclohexane ait globalement un rapport pondéral cyclohexane/acide acétique compris entre 1/1 et 50/1 et de préférence entre 2/1 et 15/1.

18. Procédé selon la revendication 17, caractérisé en ce que l'extraction est réalisée en une ou plusieurs fois ou dans le cadre d'un procédé en continu par les techniques industrielles habituelles et qu'elle est conduite à une température allant jusqu'à la température d'ébullition du ou des solvants utilisés, de préférence entre 10°C et 80°C et plus préférentiellement entre 50°C et 80°C.

19. Procédé selon l'une des revendications 17 ou 18, caractérisé en ce que l'opération d'extraction conduit d'une part à une solution qui contient au moins une partie des acides glutarique et succinique, ainsi que des quantités résiduelles d'autres sous-produits et d'autre part à un mélange contenant essentiellement le catalyseur au cobalt, ce mélange pouvant être séparé par décantation.

20. Procédé selon la revendication 19, caractérisé en ce que le catalyseur au cobalt séparé est recyclé dans une nouvelle réaction d'oxydation du cyclohexane, éventuellement après l'ajout d'un appoint pour compenser les pertes subies au cours des différents traitements du mélange réactionnel issu de l'oxydation du cyclohexane.

21. Procédé continu d'oxydation du cyclohexane en acide adipique, à l'aide d'un gaz contenant de l'oxygène, en milieu liquide comprenant un acide carboxylique comme solvant et en présence d'un catalyseur contenant au moins du cobalt, caractérisé en ce qu'il comporte les étapes suivantes :
a) l'oxydation proprement dite du cyclohexane en acide adipique,
b) le mélange réactionnel provenant de l'oxydation du cyclohexane en acide adipique est traité pour éliminer les composés les plus volatils, après éventuellement la cristallisation et la séparation d'au moins une partie de l'acide adipique qu'il contient,
c) le résidu obtenu est extrait à l'aide d'un solvant, choisi parmi les cétones, les alcools, les esters, leurs différents mélanges ou les mélanges d'hydrocarbure et d'acide carboxylique, solvant capable de dissoudre la totalité ou une grosse partie des diacides que contient ledit résidu,
d) le résidu d'extraction ainsi obtenu, contenant la plus grosse partie du catalyseur au cobalt, est utilisé dans une nouvelle opération d'oxydation du cyclohexane a).

22. Procédé continu d'oxydation du cyclohexane en acide adipique, à l'aide d'un gaz contenant de l'oxygène , en milieu liquide comprenant de l'acide acétique comme solvant et en présence d'un catalyseur contenant au moins du cobalt, caractérisé en ce qu'il comporte les étapes suivantes selon l'une des revendications 5 à 20 :
a) l'oxydation proprement dite du cyclohexane en acide adipique,
b)
soit: b1) le refroidissement du mélange réactionnel final brut, éventuellement après une concentration, afin de faire cristalliser au moins une partie de l'acide adipique, la séparation dudit acide adipique par filtration ou centrifugation, puis si nécessaire la séparation par décantation du filtrat ou du centrifugat obtenu en une phase cyclohexanique et une phase acétique,
soit : b2) le soutirage à chaud du mélange réactionnel final brut, ledit mélange réactionnel étant biphasique ou monophasique, son éventuelle séparation par décantation en une phase cyclohexanique et une phase acétique, le refroidissement de la phase acétique décantée ou le cas échéant de la phase acétique unique, afin de faire cristalliser au moins une partie de l'acide adipique, la séparation par filtration ou centrifugation dudit acide adipique et de la phase acétique,
soit : b3) l'élimination de l'eau du mélange réactionnel pendant l'étape a) d'oxydation, par distilation de l'azéotrope cyclohexane/eau et réintroduction éventuelle dans le réacteur du cyclohexane distillé, puis traitement du mélange réactionnel final brut selon la variante b1), ledit traitement conduisant à une phase acétique unique,
c) la concentration de la phase acétique pour en éliminer au moins la majeure partie de l'eau, si cela n'a pas été fait dans la variant b3),
d) l'extraction de la phase acétique à l'aide de cyclohexane ou de mélange cyclohexane/acide acétique en quantité telle que dans l'ensemble cyclohexane/phase acétique le rapport pondéral global cyclohexane/acide acétique soit compris entre 1/1 et 50/1 et de préférence entre 2/1 et 15/1,
e) la séparation d'une solution cyclohexanique contenant au moins une partie des acides glutarique et succinique, d'une part, et d'un mélange contenant essentiellement le catalyseur au cobalt, d'autre part,
f) le recyclage du mélange contenant le catalyseur au cobalt dans une nouvelle réaction d'oxydation a).

## Patentansprüche

1. Verfahren zur Wiederverwendung eines Cobalt enthaltenden Katalysators in einer Reaktion der direkten Oxidation von Cyclohexan zu Adipinsäure, dadurch gekennzeichnet, daß es eine Stufe der Behandlung der bei der Oxidation von Cyclohexan zu Adipinsäure erhaltenen Reaktionsmischung umfaßt, die in einer Extraktion von mindestens einem Teil der bei der Reaktion gebildeten Glutarsäure und Bernsteinsäure besteht.

2. Verfahren nach Anspruch 1 zur Wiederverwendung eines Cobalt enthaltenden Katalysators in einer Reaktion der direkten Oxidation von Cyclohexan zu Adipinsäure in einem Lösungsmittel, das mindestens eine aliphatische Carbonsäure umfaßt, die nur primäre oder sekundäre Wasserstoffatome besitzt, durch ein Sauerstoff enthaltendes Gas, wobei das genannte Verfahren zur Wiederverwendung dadurch gekennzeichnet ist, daß
- die aus einer früheren Operation der Oxidation von Cyclohexan zu Adipinsäure stammende Reaktionsmischung durch Destillation behandelt wird, um die am meisten flüchtigen Verbindungen zu entfernen, gegebenenfalls nach der Kristallisation und der Abtrennung von mindestens einem Teil der sie enthaltenden Adipinsäure,
- der erhaltene Rückstand mit Hilfe eines Lösungsmittels extrahiert wird, ausgewählt unter den Ketonen, den Alkoholen, den Estern, ihren verschiedenen Mischungen oder den Mischungen von Kohlenwasserstoff und Carbonsäure, wobei das Lösungsmittel fähig ist, die Gesamtheit oder einen großen Teil der Disäuren zu lösen, die der genannte Rückstand enthält,
- der auf diese Weise erhaltene Extraktionsrückstand, der den größten Teil des Cobalt-Katalysators enthält, nach der Zugabe der notwendigen Ergänzungen an Cyclohexan, an Carbonsäure und gegebenenfalls an Cobalt-Katalysator, in einer neuen Operation der Oxidation von Cyclohexan zu Adipinsäure verwendet wird.

3. Verfahren nach Anspruch 2, dadurch gekennzeichnet, daß die Behandlung der Reaktionsmischung durch Destillation der am meisten flüchtigen Verbindungen unter atmosphärischem Druck oder unter reduziertem Druck erfolgt, die insbesondere aus nicht umgesetztem Cyclohexan, bei der Oxidationsreaktion als Lösungsmittel dienender Carbonsäure, gebildetem Wasser und einigen Zwischenverbindungen wie Cyclohexanol und Cyclohexanon bestehen.

4. Verfahren nach Anspruch 2, dadurch gekennzeichnet, daß das Lösungsmittel, das zur Extraktion des Katalysators aus dem erhaltenen Rückstand dient, unter Aceton, Methylethylketon, Cyclohexanon, 1-Propanol, 2-Propanol, 1-Butanol, 2-Butanol, tert.-Butanol, Cyclohexanol, den von den vorstehend genannten Alkoholen mit aliphatischen Carbonsäuren, die nur primäre oder sekundäre Wasserstoffatome besitzen wie denjenigen, die bei der Oxidationsreaktion von Cyclohexan eingesetzt wurden, abgeleiteten Estern, den Mischungen von mehreren dieser Extraktions-Lösungsmittel und den Mischungen von aliphatischen oder cycloaliphatischen Kohlenwasserstoffen und Carbonsäuren ausgewählt wird.

5. Verfahren nach Anspruch 1 zur Wiederverwendung eines Cobalt enthaltenden Katalysators in einer Reaktion der direkten Oxidation von Cyclohexan zu Adipinsäure durch ein Sauerstoff enthaltendes Gas, wobei das genannte Verfahren zur Wiederverwendung dadurch gekennzeichnet ist, daß
- die aus einer früheren Operation der Oxidation von Cyclohexan zu Adipinsäure stammende Reaktionsmischung, von der mindestens ein Teil der Zwischenprodukte der Oxidation wie insbesondere Cyclohexanol und Cyclohexanon, Carbonsäure als Lösungsmittel und Wasser abgetrennt wurde, und von der mindestens ein Teil der gebildeten Adipinsäure durch Kristallisation gewonnen wurde, mindestens einer Extraktion mit Hilfe von mindestens einem Co-Lösungsmittel oder einer Mischung, die ein Co-Lösungsmittel und eine Carbonsäure umfaßt, unterzogen wird,
- man einerseits eine Mischung, die mindestens einen Teil des Cobalt-Katalysators, einen Teil der Carbonsäure und gegebenenfalls restliche Mengen von anderen Verbindungen enthält, und andererseits eine Lösung, die das Co-Lösungsmittel und mindestens einen Teil der bei der Oxidationsreaktion gebildeten Glutarsäure und Bernsteinsäure sowie die Carbonsäure enthält, abtrennt,
- die Mischung, die mindestens einen Teil des Cobalt-Katalysators enthält, gegebenenfalls nach der Zugabe von zusätzlichem Cobalt-Katalysator, in einer neuen Operation der Oxidation von Cyclohexan zu Adipinsäure verwendet wird.

6. Verfahren nach Anspruch 5, dadurch gekennzeichnet, daß das eingesetzte Co-Lösungsmittel unter den aliphatischen und cycloaliphatischen Kohlenwasserstoffen, den Ketonen und den Alkoholen ausgewählt wird und vorzugsweise Cyclohexan ist.

7. Verfahren nach einem der Ansprüche 2 bis 5, dadurch gekennzeichnet, daß die verwendete aliphatische Carbonsäure die Essigsäure ist.

8. Verfahren nach einem der Ansprüche 5 bis 7, dadurch gekennzeichnet, daß die rohe Reaktionsmischung einer Abkühlung auf eine Temperatur von 16 °C bis 30 °C unterzogen wird, um die Kristallisation von mindestens einem Teil der gebildeten Adipinsäure herbeizuführen, indem man sie dann entweder auf ein triphasisches Milieu, das eine im wesentlichen aus Adipinsäure bestehende feste Phase, eine obere flüssige Cyclohexan-Phase und eine untere flüssige Essigsäure-Phase umfaßt, oder auf ein biphasisches Milieu bringt, das eine im wesentlichen aus Adipinsäure bestehende feste Phase, und eine Essigsäure-Phase umfaßt, und anschließend nach der Filtration oder dem Zentrifugieren des Feststoffes gegebenenfalls die Trennung der zwei flüssigen Phasen durch Dekantieren vorgenommen wird.

9. Verfahren nach einem der Ansprüche 5 oder 8, dadurch gekennzeichnet, daß man vor der Operation der Kristallisation der Adipinsäure eine Konzentration der Reaktionsmischung durchführt.

10. Verfahren nach einem der Ansprüche 5 bis 7, dadurch gekennzeichnet, daß man die fertige rohe Reaktionsmischung in der Wärme abzieht, wobei die genannte Reaktionsmischung dann biphasisch oder monophasisch ist, daß man, wenn erforderlich, die Trennung durch Dekantieren der zwei flüssigen Phasen in eine obere Cyclohexan-Phase und eine untere Essigsäure-Phase vornimmt, daß man die genannte untere Essigsäure-Phase oder die einzige Essigsäure-Phase auf eine Temperatur von 16 °C bis 30 °C abkühlt, um die Kristallisation von mindestens einem Teil der gebildeten Adipinsäure herbeizuführen, die dann anschließend durch Filtration oder Zentrifugieren von der Essigsäure-Phase abgetrennt wird.

11. Verfahren nach einem der Ansprüche 8 oder 10, dadurch gekennzeichnet, daß man die Adipinsäure durch Rekristallisation in einem geeigneten Lösungsmittel wie Essigsäure oder Wasser reinigt, das gegebenenfalls anschließend wieder zu der vorstehend erhaltenen Essigsäure-Phase gegeben wird.

12. Verfahren nach einem der Ansprüche 2 bis 5, dadurch gekennzeichnet, daß man während der Oxidationsreaktion eine azeotrope Mischung Wasser/Cyclohexan destilliert und man anschließend das Cyclohexan nach dem Dekantieren dieser Mischung wieder in den Reaktor einträgt sowie die fertige rohe Reaktionsmischung behandelt.

13. Verfahren nach einem der Ansprüche 7 bis 12, dadurch gekennzeichnet, daß die bei der Behandlung der fertigen rohen Reaktionsmischung erhaltene Essigsäure-Phase, entweder so wie sie nach dem Dekantieren erhalten wurde oder vorzugsweise, nachdem sie durch Erhitzen unter reduziertem Druck auf eine Temperatur von 30 °C bis 80 °C konzentriert wurde, einer Extraktion durch Cyclohexan unterzogen wird.

14. Verfahren nach Anspruch 13, dadurch gekennzeichnet, daß das Konzentrieren der Essigsäure-Phase diese auf ein Volumen verringert, das 80 % bis 10 % ihres Ausgangsvolumens darstellt.

15. Verfahren nach Anspruch 13, dadurch gekennzeichnet, daß man die Essigsäure-Phase bis zur Trockne konzentriert, das heißt, daß man die Gesamtheit der sie enthaltenden Essigsäure entfernt.

16. Verfahren nach einem der Ansprüche 13 bis 15, dadurch gekennzeichnet, daß die von der Essigsäure-Phase bei ihrer Konzentration getrennten Verbindungen, entweder insgesamt oder nach Abtrennung von mindestens einem Teil des sie enthaltenden Wassers, in die Stufe der Oxidation des Cyclohexans zurückgeführt werden.

17. Verfahren nach einem der Ansprüche 7 bis 16, dadurch gekennzeichnet, daß die Extraktion der konzentrierten oder nicht konzentrierten Essigsäure-Phase entweder nur mit Cyclohexan allein oder mit Mischungen Cyclohexan/Essigsäure durchgeführt wird, damit die Gesamtheit Essigsäure-Phase, die der Extraktion unterzogen wurde/Cyclohexan global ein Gewichtsverhältnis Cyclohexan/Essigsäure zwischen 1/1 und 50/1, vorzugsweise zwischen 2/1 und 15/1 aufweist.

18. Verfahren nach Anspruch 17, dadurch gekennzeichnet, daß die Extraktion einmal oder mehrmals oder im Rahmen eines kontinuierlichen Verfahrens nach üblichen industriellen Techniken durchgeführt wird und daß sie bei einer Temperatur erfolgt, die bis zur Siedetemperatur des oder der verwendeten Lösungsmittel(s) reicht, vorzugsweise zwischen 10 °C und 80 °C und noch bevorzugter zwischen 50 °C und 80 °C.

19. Verfahren nach einem der Ansprüche 17 oder 18, dadurch gekennzeichnet, daß die Operation der Extraktion einerseits zu einer Lösung führt, die mindestens einen Teil der Glutarsäure und der Bernsteinsäure sowie restliche Mengen von anderen Nebenprodukten enthält, und andererseits zu einer Mischung, die im wesentlichen den Cobalt-Katalysator enthält, wobei diese Mischung durch Dekantieren getrennt werden kann.

20. Verfahren nach Anspruch 19, dadurch gekennzeichnet, daß der abgetrennte Cobalt-Katalysator in eine neue Reaktion der Oxidation von Cyclohexan zurückgeführt wird, gegebenenfalls nach der Zugabe eines Zusatzes, um die im Verlauf der unterschiedlichen Behandlungen der von der Oxidation des Cyclohexans stammenden Reaktionsmischung erlittenen Verluste zu kompensieren.

21. Kontinuierliches Verfahren zur Oxidation von Cyclohexan zu Adipinsäure mit Hilfe eines Sauerstoff enthaltenden Gases im flüssigen Medium, das eine Carbonsäure als Lösungsmittel umfaßt, und in Anwesenheit eines mindestens Cobalt enthaltenden Katalysators, dadurch gekennzeichnet, daß es die folgenden Stufen umfaßt:
a) eigentliche Oxidation von Cyclohexan zu Adipinsäure,
b) die von der Oxidation des Cyclohexans zu Adipinsäure stammende Reaktionsmischung wird behandelt, um die am meisten flüchtigen Verbindungen zu entfernen, gegebenenfalls nach der Kristallisation und der Abtrennung von mindestens einem Teil der sie enthaltenden Adipinsäure,
c) der erhaltene Rückstand wird mit Hilfe eines Lösungsmittels extrahiert, ausgewählt unter den Ketonen, den Alkoholen, den Estern, ihren verschiedenen Mischungen oder den Mischungen von Kohlenwasserstoff und Carbonsäure, wobei das Lösungsmittel fähig ist, die Gesamtheit oder einen großen Teil der Disäuren zu lösen, die der genannte Rückstand enthält,
d) der auf diese Weise erhaltene Extraktionsrückstand, der den größten Teil des Cobalt-Katalysators enthält, wird in einer neuen Operation der Oxidation von Cyclohexan a) verwendet.

22. Kontinuierliches Verfahren zur Oxidation von Cyclohexan zu Adipinsäure mit Hilfe eines Sauerstoff enthaltenden Gases im flüssigen Medium, das Essigsäure als Lösungsmittel umfaßt, und in Anwesenheit eines mindestens Cobalt enthaltenden Katalysators, dadurch gekennzeichnet, daß es die folgenden Stufen nach einem der Ansprüche 5 bis 20 umfaßt:
a) eigentliche Oxidation von Cyclohexan zu Adipinsäure,
b) entweder:
b1) die Abkühlung der fertigen rohen Reaktionsmischung, gegebenenfalls nach einer Konzentration, um mindestens einen Teil der Adipinsäure zu kristallisieren, die Abtrennung der genannten Adipinsäure durch Filtration oder Zentrifugieren, anschließend, wenn erforderlich, die Trennung durch Dekantieren des erhaltenen Filtrates oder Zentrifugates in eine Cyclohexan-Phase und eine Essigsäure-Phase, oder:
b2) das Abziehen der fertigen rohen Reaktionsmischung in der Wärme, wobei die genannte Reaktionsmischung dann biphasisch oder monophasisch ist, ihre eventuelle Trennung durch Dekantieren in eine Cyclohexan-Phase und eine Essigsäure-Phase, das Abkühlen der dekantierten Essigsäure-Phase oder gegebenenfalls der einzigen Essigsäure-Phase, um mindestens einen Teil der Adipinsäure zu kristallisieren, die Trennung der genannten Adipinsäure und der Essigsäure-Phase durch Filtration oder Zentrifugieren, oder:
b3) die Entfernung des Wassers aus der Reaktionsmischung während Stufe a) der Oxidation durch azeotrope Destillation Cyclohexan/Wasser und eventuelles Wiedereintragen des destillierten Cyclohexans in den Reaktor, anschließende Behandlung der fertigen rohen Reaktionsmischung gemäß Variante b1), wobei die genannte Behandlung zu einer einzigen Essigsäure-Phase führt,
c) die Konzentration der Essigsäure-Phase, um mindestens den überwiegenden Teil des Wassers zu entfernen, wenn dies nicht bereits in der Variante b3) erfolgte,
d) die Extraktion der Essigsäure-Phase mit Hilfe von Cyclohexan oder einer Mischung Cyclohexan/Essigsäure in einer solchen Menge, daß in der Gesamtheit Cyclohexan/Essigsäure-Phase das globale Gewichtsverhältnis Cyclohexan/Essigsäure zwischen 1/1 und 50/1, vorzugsweise zwischen 2/1 und 15/1 beträgt,
e) die Abtrennung einerseits einer Lösung von Cyclohexan, die mindestens einen Teil der Glutarsäure und der Bernsteinsäure enthält, und andererseits einer Mischung, die im wesentlichen den Cobalt-Katalysator enthält,
f) die Rückführung der den Cobalt-Katalysator enthaltenden Mischung in eine neue Oxidationsreaktion a).

## Claims

1. Process for recycling a catalyst containing cobalt, in a reaction for the direct oxidation of cyclohexane to adipic acid, characterized in that it includes a step for the treatment of the reaction mixture obtained during the oxidation of the cyclohexane to adipic acid, consisting of an extraction of at least some of the glutaric acid and succinic acid which are formed in the reaction.

2. Process according to Claim 1 for recycling a catalyst containing cobalt, in a reaction for the direct oxidation of cyclohexane to adipic acid, in a solvent comprising at least one aliphatic carboxylic acid having only primary or secondary hydrogen atoms, by a gas containing oxygen, the said recycling process being characterized:
- in that the reaction mixture derived from a prior operation for the oxidation of the cyclohexane to adipic acid is treated by distillation in order to remove the more volatile compounds, optionally after the crystallization and separation of at least some of the adipic acid which it contains,
- in that the residue obtained is extracted using a solvent, chosen from ketones, alcohols, esters, various mixtures thereof or mixtures of hydrocarbon and carboxylic acid, this solvent being capable of dissolving all or a large part of the diacids contained in the said residue,
- in that the extraction residue thus obtained, containing the larger part of the cobalt catalyst, is used in a new operation for the oxidation of cyclohexane to adipic acid, after addition of the necessary complements of cyclohexane, of carboxylic acid and, where appropriate, of cobalt catalyst.

3. Process according to Claim 2, characterized in that the reaction mixture is treated by distillation, at atmospheric pressure or at reduced pressure, of the more volatile compounds which are especially the unreacted cyclohexane, the carboxylic acid serving as solvent in the oxidation reaction, the water formed and certain intermediate compounds such as cyclohexanol and cyclohexanone.

4. Process according to Claim 2, characterized in that the solvent which serves for extraction of the catalyst from the residue obtained is chosen from acetone, methyl ethyl ketone, cyclohexanone, 1-propanol, 2-propanol, 1-butanol, 2-butanol, tert-butanol, cyclohexanol, esters derived from the alcohols mentioned above with aliphatic carboxylic acids having only primary or secondary hydrogen atoms, such as those which are used in the cyclohexane oxidation reaction, mixtures of several of these extraction solvents, and mixtures of aliphatic or cycloaliphatic hydrocarbons and carboxylic acids.

5. Process according to Claim 1 for recycling a catalyst containing cobalt, in a reaction for the direct oxidation of cyclohexane to adipic acid, by a gas containing oxygen, the said recycling process being characterized:
- in that the reaction mixture, obtained from a prior operation of oxidation of the cyclohexane to adipic acid, from which mixture at least some of the intermediate oxidation products, especially such as cyclohexanol and cyclohexanone, some carboxylic acid solvent and water has been separated and from which mixture at least some of the adipic acid formed has been recovered by crystallization, is subjected to at least one extraction using at least one cosolvent or using a mixture comprising a cosolvent and a carboxylic acid,
- in that there are separated, on the one hand, a mixture containing at least some of the cobalt catalyst, some of the carboxylic acid and possibly residual amounts of other compounds and, on the other hand, a solution containing the cosolvent and at least some of the glutaric acid and succinic acid which are formed in the oxidation reaction, as well as some carboxylic acid,
- and in that the mixture containing at least some of the cobalt catalyst is used in a new operation for the oxidation of cyclohexane to adipic acid, optionally after the addition of extra cobalt catalyst.

6. Process according to Claim 5, characterized in that the cosolvent used is chosen from aliphatic and cycloaliphatic hydrocarbons, ketones and alcohols, and is preferably cyclohexane.

7. Process according to one of Claims 2 to 5, characterized in that the aliphatic carboxylic acid used is acetic acid.

8. Process according to one of Claims 5 to 7, characterized in that the crude reaction mixture is subjected to cooling to a temperature of 16°C to 30°C to bring about the crystallization of at least some of the adipic acid formed, thus giving either a three-phase medium comprising a solid phase essentially consisting of adipic acid, an upper cyclohexane liquid phase and a lower acetic liquid phase, or giving a two-phase medium comprising a solid phase essentially consisting of adipic acid and an acetic phase, followed, if need be, after filtration or centrifugation of the solid, by separation of the two liquid phases after settling.

9. Process according to either of Claims 5 and 8, characterized in that the reaction mixture is concentrated prior to the adipic acid crystallization operation.

10. Process according to one of Claims 5 to 7, characterized in that the final crude reaction mixture is removed while hot, the said reaction mixture then being a two-phase or monophase mixture, two liquid phases: an upper cyclohexane phase and a lower acetic liquid phase are separated after settling, if necessary, and the said lower acetic phase or the single acetic phase is cooled to a temperature of 16°C to 30°C to bring about the crystallization of at least some of the adipic acid formed, which is then separated out by filtration or centrifugation of the acetic phase.

11. Process according to either of Claims 8 and 10, characterized in that the adipic acid is purified by recrystallization from a suitable solvent, such as acetic acid or water, which is then optionally added to the acetic phase obtained above.

12. Process according to one of Claims 2 to 5, characterized in that a water/cyclohexane azeotropic mixture is distilled off during the oxidation reaction, and the cyclohexane is then reintroduced into the reactor after separation of this mixture by settling and the final crude reaction mixture is treated.

13. Process according to one of Claims 7 to 12, characterized in that the acetic phase, obtained in the treatment of the final crude reaction mixture, is subjected to an extraction with cyclohexane, either such as it is obtained after separation by settling, or preferably after having been concentrated by heating to a temperature of 30°C to 80°C at reduced pressure.

14. Process according to Claim 13, characterized in that concentration of the acetic phase reduces this phase to a volume representing from 80 % to 10 % of its initial volume.

15. Process according to Claim 13, characterized in that the acetic phase is concentrated to dryness, that is to say that all of the acetic acid which it contains is removed.

16. Process according to one of Claims 13 to 15, characterized in that the compounds separated from the acetic phase during concentration thereof are recycled in the cyclohexane oxidation step, either in total or after separation of at least some of the water which they contain.

17. Process according to one of Claims 7 to 16, characterized in that the concentrated or non-concentrated acetic phase is extracted either with cyclohexane alone or with cyclohexane/acetic acid mixtures, so that the acetic phase subjected to the extraction/cyclohexane mixture has an overall cyclohexane/acetic acid weight ratio between 1/1 and 50/1 and preferably between 2/1 and 15/1.

18. Process according to Claim 17, characterized in that the extraction is performed one or more times or, within the context of a continuous process, by the usual industrial techniques and in that it is carried out at a temperature ranging up to the boiling point of the solvent or solvents used, preferably between 10°C and 80°C and more preferably between 50°C and 80°C.

19. Process according to either of Claims 17 and 18, characterized in that the extraction operation gives, on the one hand, a solution which contains at least some of the glutaric acid and succinic acid, as well as residual amounts of other by-products, and, on the other hand, a mixture essentially containing the cobalt catalyst, it being possible for this mixture to be separated by settling.

20. Process according to Claim 19, characterized in that the separated cobalt catalyst is recycled in a new cyclohexane oxidation reaction, optionally after a further addition to compensate for the losses suffered during the various treatments of the reaction mixture obtained from the cyclohexane oxidation.

21. Continuous process for the oxidation of cyclohexane to adipic acid, using a gas containing oxygen, in a liquid medium comprising a carboxylic acid as solvent and in the presence of a catalyst containing at least cobalt, characterized in that it includes the following steps:
a) the actual oxidation of the cyclohexane to adipic acid,
b) the reaction mixture derived from the oxidation of the cyclohexane to adipic acid is treated in order to remove the more volatile compounds, optionally after crystallization and separation of at least some of the adipic acid which it contains,
c) the residue obtained is extracted using a solvent chosen from ketones, alcohols, esters, various mixtures thereof or mixtures of hydrocarbon and carboxylic acid, which solvent is capable of dissolving all or a large part of the diacids contained in the said residue,
d) the extraction residue thus obtained, containing the larger part of the cobalt catalyst, is used in a new cyclohexane oxidation operation a).

22. Continuous process for the oxidation of cyclohexane to adipic acid, using a gas containing oxygen, in a liquid medium comprising acetic acid as solvent and in the presence of a catalyst containing at least cobalt, characterized in that it includes the following steps according to one of Claims 5 to 20:
a) the actual oxidation of the cyclohexane to adipic acid,
b) either:
b1) cooling of the crude final reaction mixture, optionally after concentrating, in order to crystallize at least some of the adipic acid, separation of the said adipic acid by filtration or centrifugation and then, if necessary, separation by settling of the filtrate or of the centrifugate obtained into a cyclohexane phase and an acetic phase,
or: b2) removal of the crude final reaction mixture while hot, the said reaction mixture being a two-phase or a monophase mixture, its possible separation by settling into a cyclohexane phase and an acetic phase, cooling of the acetic phase which has been separated by settling or, where appropriate, of the single acetic phase so as to crystallize at least some of the adipic acid, and separation by filtration or centrifugation of the said adipic acid and of the acetic phase,
or: b3) removal of the water from the reaction mixture during the oxidation step a), by distillation of the cyclohexane/water azeotrope and optional reintroduction of the distilled cyclohexane into the reactor, followed by treatment of the crude final reaction mixture according to the variant b1), the said treatment giving a single acetic phase
c) concentration of the acetic phase in order to remove therefrom at least the majority of the water, if this has not been done in the variant b3),
d) extraction of the acetic phase using cyclohexane or a cyclohexane/acetic acid mixture in an amount such that, in the cyclohexane/acetic phase mixture, the overall cyclohexane/acetic acid weight ratio is between 1/1 and 50/1 and preferably between 2/1 and 15/1,
e) separation of a cyclohexane solution containing at least some of the glutaric acid and succinic acid, on the one hand, and of a mixture essentially containing the cobalt catalyst, on the other hand,
f) recycling of the mixture containing the cobalt catalyst in a new oxidation reaction a).
